# EUROPÄISCHE PATENTSCHRIFT

(11) **EP 2 694 305 B1**
(45) Veröffentlichungstag und Bekanntmachung des Hinweises auf die Patenterteilung: **22.02.2017**
(21) Anmeldenummer: 11793647.6
(22) Anmeldetag: 30.11.2011
(51) Int. Cl.: B60H 3/00, B60R 7/08

(54) **BEDUFTUNGSVORRICHTUNG FÜR EIN FAHRZEUG**
DEODORIZATION DEVICE FOR A VEHICLE
DIFFUSEUR DE PARFUM POUR UN VÉHICULE

(30) Priorität: 29.03.2011 DE 102011001637
(43) Veröffentlichungstag der Anmeldung: 12.02.2014
(73) Patentinhaber: Supair-Tel AG, 8152 Glattbrugg (CH)
(72) Erfinder: GUGGENHEIM, Rudolf, CH-8702 Zollikon (CH); KELLER, Beat, CH-8046 Zürich (CH)
(74) Vertreter: Fischer, Britta Ruth
(86) Internationale Anmeldenummer: PCT/CH2011/000290
(87) Internationale Veröffentlichungsnummer: WO 2012/129709

(56) Entgegenhaltungen:
- EP-A1- 1 902 736
- DE-A1-102009 006 192
- JP-A- 2000 185 588
- US-A1- 2005 127 538
- US-A1- 2009 114 736

## Beschreibung

### Technisches Gebiet

Die Erfindung betrifft eine Beduftungsvorrichtung für ein Fahrzeug gemäss Oberbegriff des Anspruchs 1, wie offenbart in US2009/0114736.

Der Begriff "Fahrzeug" umfasst hierbei sowohl Landfahrzeuge wie Personenkraftwagen und Lastkraftwagen als auch Luftfahrzeuge.

### Stand der Technik

Aus der Offenlegungsschrift DE 10 2009 006 192 A1 ist eine Beduftungsvorrichtung für den Innenraum eines Fahrzeugs bekannt. Die Beduftungsvorrichtung weist einen Flakon für einen Duftstoff auf, welcher innerhalb einer Aufnahmevorrichtung mit einer Lufteinströmöffnung und einer Luftausströmöffnung angeordnet ist. Die Beduftungsvorrichtung kann mittels einer Verrohrung auf einer Fondmitteldüse im Innenraum eines Fahrzeugs angeordnet werden. Die Verrohrung ist mit der Lufteinströmöffnung verbunden, sodass ein von einer Membranpumpe geförderter Luftstrom mit vorkonditionierter und gereinigter Luft aus dem Innenraum des Fahrzeugs durch die Verrohrung und die Lufteinströmöffnung in das Innere der Aufnahmevorrichtung und weiter um den Flakon herum in einen oberen Bereich der Aufnahmevorrichtung strömt, wo er über Öffnungen in das Innere des Flakons gelangt und die Oberfläche des Duftstoffs berührt. Über ein Ausströmrohr und die Luftausströmöffnung der Aufnahmevorrichtung strömt der nun mit Duftmolekülen angereicherte Luftstrom in den Innenraum des Fahrzeugs.

### Darstellung der Erfindung

Es ist Aufgabe der Erfindung, eine Beduftungsvorrichtung für ein Fahrzeug bereitzustellen, welche einfach aufgebaut ist, welche benutzerfreundlich ist, indem sie sich einfach und stabil in einem Innenraum eines Fahrzeugs anbringen lässt, und welche eine effiziente Beduftung des Innenraums eines Fahrzeugs ermöglicht.

Diese Aufgabe wird durch eine Beduftungsvorrichtung mit den Merkmalen des Anspruchs 1 gelöst.

Die erfindungsgemässe Beduftungsvorrichtung umfasst einen Behälter für einen Duftstoff mit einer Haltevorrichtung zum Anbringen der Beduftungsvorrichtung an einem Lüftungsschlitz im Innenraum eines Fahrzeugs, insbesondere an einem Lüftungsschlitz einer Fondmitteldüse eines Fahrzeugs. Der Behälter für den Duftstoff weist eine oder mehrere Öffnungen auf. Die Haltevorrichtung umfasst wenigstens ein Klemmteil aus elastischem Material, das einen elliptischen oder kreisförmigen Querschnitt aufweist. Das Klemmteil ist derart ausgestaltet, dass es haltend in einen Lüftungsschlitz eines Fahrzeugs eingebracht werden kann.

Die Querrichtung des Klemmteils ist als die Richtung definiert, die senkrecht zu der Richtung verläuft, in die sich das Klemmteil erstreckt. Die Richtung, in die sich das Klemmteil erstreckt, entspricht dessen Längsrichtung. Eine Ellipse ist vorliegend als ovale geschlossene Kurve definiert (http://de.wikipedia.org/wiki/Ellipse).

Das wenigstens eine Klemmteil der erfindungsgemässen Beduftungsvorrichtung weist gemäss bevorzugter Ausgestaltung eine Hüllform auf, die der Form eines elliptischen Paraboloids, eines Kegels oder eines Zylinders entspricht, wobei der Extremwert des Paraboloids bzw. die Spitze des Kegels von dem Behälter für den Duftstoff wegweist. Unter einer Hüllform wird die dreidimensionale Variante einer Hüllkurve bzw. Einhüllenden verstanden (http://de.wikipedia.org/wiki/Einhüllende). Unter dem Extremwert ist das Maximum des Paraboloids in Längsrichtung des Klemmteils zu verstehen.

Das Klemmteil ist vorzugsweise aus bzw. umfasst als Material vorzugsweise Silikon, Gummi und/oder ein thermoplastisches Elastomer, wobei das Material des Klemmteils bevorzugt eine Härte im Bereich von 30 Shore-A bis 90 Shore-A hat.

Die erfindungsgemässe Beduftungsvorrichtung lässt sich mittels des wenigstens einen Klemmteils einfach in einen Lüftungsschlitz im Innenraum eines Fahrzeugs einbringen und wird von einem solchen auch bei Bewegungen und Erschütterungen des Fahrzeugs sicher und stabil gehalten. Indem die Beduftungsvorrichtung an einem Lüftungsschlitz im Innenraum eines Fahrzeugs angebracht wird, kann durch den Lüftungsschlitz austretende Luft über die eine oder mehreren in dem Behälter für den Duftstoff vorgesehenen Öffnungen mit dem Duftstoff in Berührung kommen und auf diese Weise mit dessen Duftmolekülen angereichert werden, sodass mit Duftmolekülen angereicherte Luft in den Fahrzeuginnenraum eintritt und diesen effizient beduftet.

### Kurze Beschreibung der Zeichnungen

Weitere vorteilhafte Ausgestaltungen der Erfindung ergeben sich aus den abhängigen Ansprüchen und den anhand der Zeichnungen nachfolgend dargestellten Ausführungsbeispielen. Es zeigen:
Fig. 1 eine perspektivische Darstellung einer erfindungsgemässen Beduftungsvorrichtung,
Fig. 2 eine Draufsicht auf eine erfindungsgemässe Beduftungsvorrichtung,
Fig. 3 eine Schnittdarstellung einer erfindungsgemässen Beduftungsvorrichtung entlang der Schnittlinie A-A' in Figur 2,
Fig. 4 eine Vorderansicht einer erfindungsgemässen Beduftungsvorrichtung,
Fig. 5 eine Rückansicht einer erfindungsgemässen Beduftungsvorrichtung,
Fig. 6 eine Seitenansicht einer erfindungsgemässen Beduftungsvorrichtung,
Fig. 7 eine Explosionsdarstellung einer erfindungsgemässen Beduftungsvorrichtung,
Fig. 8 eine Darstellung eines Klemmteils einer erfindungsgemässen Beduftungsvorrichtung als Längsschnitt (Figur 8a)), als Unteransicht (Figur 8b)) und in Perspektive (Figur 8c)),
Fig. 9 eine Darstellung eines mit Noppen versehen Klemmteils einer erfindungsgemässen Beduftungsvorrichtung als Unteransicht (Figur 9a)), als Längsschnitt (Figur 9b), obere Hälfte) und als Seitenansicht (Figur 9b), untere Hälfte),
Fig. 10 eine Darstellung eines mit Stützgliedern versehenen Klemmteils einer erfindungsgemässen Beduftungsvorrichtung als Längsschnitt (Figur 10a), obere Hälfte), als Seitenansicht (Figur 10a), untere Hälfte), und als Unteransicht (Figur 10b)) in einer ersten Ausgestaltung,
Fig. 11 eine Darstellung eines mit Stützgliedern versehenen Klemmteils einer erfindungsgemässen Beduftungsvorrichtung als Längsschnitt (Figur 11a), obere Hälfte), als Seitenansicht (Figur 11a), untere Hälfte) und als Unteransicht (Figur 11b)) in einer zweiten Ausgestaltung,
Fig. 12 eine Darstellung eines mit Stützgliedern versehenen Klemmteils einer erfindungsgemässen Beduftungsvorrichtung als Längsschnitt (Figur 12a), obere Hälfte), als Seitenansicht (Figur 12a), untere Hälfte), und als Unteransicht (Figur 12b)) in einer dritten Ausgestaltung,
Fig. 13 eine Darstellung eines mit Stützgliedern versehenen Klemmteils einer erfindungsgemässen Beduftungsvorrichtung als Längsschnitt (Figur 13a), obere Hälfte), als Seitenansicht (Figur 13a), untere Hälfte), und als Unteransicht (Figur 13b)) in einer vierten Ausgestaltung,
Fig. 14 ein Längsschnitt (obere Hälfte) und eine Seitenansicht (untere Hälfte) eines mit sich überlappenden Schirmen versehenen Klemmteils einer erfindungsgemässen Beduftungsvorrichtung und
Fig. 15 ein Längsschnitt (obere Hälfte) und eine Seitenansicht (untere Hälfte) eines mit Lamellen versehenen Klemmteils einer Beduftungsvorrichtung.

In den Figuren bezeichnen gleiche Bezugszeichen gleiche bzw. gleich wirkende Komponenten. Dimensions- und Grössenangaben in den Figuren sind rein beispielhafter Natur und in der Einheit Millimeter angegeben.

### Weg(e) zur Ausführung der Erfindung

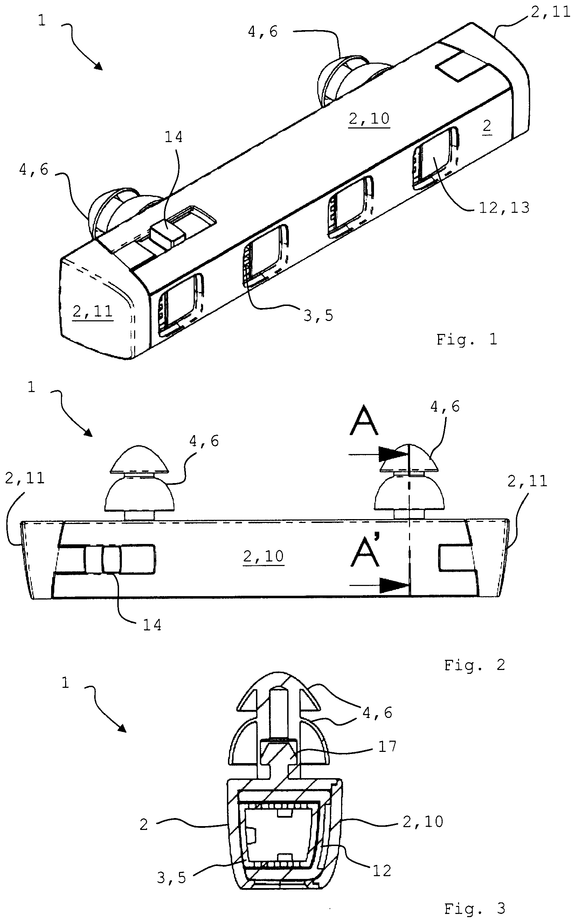

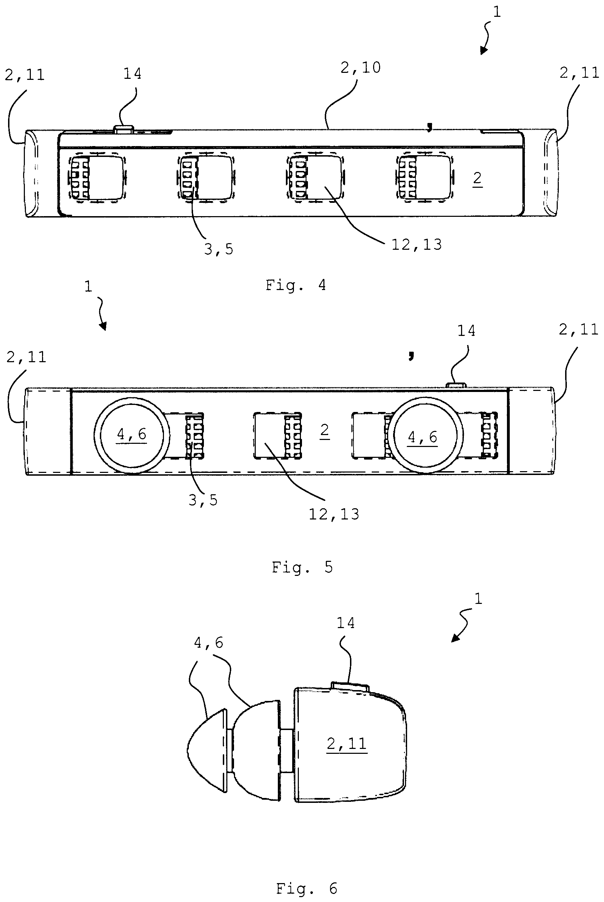

Die Figuren 1 bis 7 zeigen eine erfindungsgemässe Beduftungsvorrichtung 1 mit einem Behälter 2 für einen Duftstoff 3 und mit einer Haltevorrichtung 4 zum Anbringen der Beduftungsvorrichtung 1 an einem Lüftungsschlitz eines Fahrzeuginnenraums. Der Duftstoff 3 liegt bevorzugt in Form eines auswechselbaren Duftstifts 5 vor. Die in Figur 5 dargestellte Rückansicht der Beduftungsvorrichtung 1 entspricht im an den Lüftungsschlitz angebrachten Zustand der Beduftungsvorrichtung 1 der lüftungsschlitzseitigen Seite der Beduftungsvorrichtung 1. Die in Figur 4 dargestellte Vorderansicht der Beduftungsvorrichtung 1 entspricht der der Rückansicht gegenüberliegenden Seite der Beduftungsvorrichtung 1. Die Haltevorrichtung 4 ist an der Seite der Beduftungsvorrichtung 1 angeordnet, die im an einem Lüftungsschlitz angebrachten Zustand diesem zugewandt ist.

Die Haltevorrichtung 4 umfasst wenigstens ein Klemmteil 6 aus elastischem Material, welches einen elliptischen oder kreisförmigen Querschnitt aufweist. Das Klemmteil 4 ist derart ausgestaltet, dass es haltend in einen Lüftungsschlitz im Fahrzeuginnenraum eingebracht werden kann. Das heisst, die Dimensionen des elliptischen oder kreisförmigen Querschnitts des Klemmteils 6 sind in Abhängigkeit von den Dimensionen, insbesondere der Breite, des Lüftungsschlitzes derart gewählt, dass die Beduftungsvorrichtung 1 an den Lüftungsschlitz angebracht werden kann, indem das wenigstens eine Klemmteil 6 in den Lüftungsschlitz eingeführt/eingebracht wird und dort von diesem klemmend gehalten wird.

Der Behälter 2 weist eine, vorzugsweise mehrere Öffnungen 7 auf, die auf derselben Seite wie die Haltevorrichtung 4 vorgesehen sind. Durch diese Öffnungen 7 strömt im angebrachten Zustand der Beduftungsvorrichtung 1 Luft aus dem Lüftungsschlitz in den Behälter 2, kommt mit dem Duftstoff 3 in Kontakt und wird mit dessen Duftmolekülen angereichert. Der Behälter 2 weist vorzugsweise eine oder mehrere Öffnungen 8 auf, die der einen oder den mehreren Öffnungen 7 gegenüberliegen, über die die mit Duftmolekülen angereicherte Luft in den Fahrzeuginnenraum entweichen und diesen beduften kann. Die Öffnungen 8 weisen im angebrachten Zustand der Beduftungsvorrichtung 1 zum Fahrzeuginnenraum hin. Zusätzlich oder alternativ zu den Öffnungen 8 können auch Öffnungen an einer oder an beiden Seidenwänden des Behälters 2 und/oder an dessen Oberseite und/oder an dessen Unterseite vorgesehen sein.

Der Behälter 2 ist bevorzugt länglich ausgeführt und mit wenigstens zwei Klemmteilen 6 versehen (wie in den Figuren 1 bis 7 dargestellt), wobei je ein Klemmteil 6 in einem seitlichen Endbereich des Behälters 2 angeordnet ist. Der Behälter 2 erstreckt sich hierbei im an einen Lüftungsschlitz angebrachten Zustand entlang des Lüftungsschlitzes, wobei beide Klemmteile 6 in demselben Lüftungsschlitz angeordnet sind. Selbstverständlich ist es auch möglich, den Behälter 2 quer zu den Lüftungsschlitzen anzuordnen, wobei die Klemmteile 6 dann in unterschiedliche Lüftungsschlitze eingebracht sind.

Der Behälter für den Duftstoff kann zum Beispiel auch kreisförmig ausgeführt sein, wobei die Haltevorrichtung mit dem einen oder den mehreren Klemmteilen dann vorzugsweise mittig auf der Rückseite, d.h. der im angebrachten Zustand den Lüftungsschlitzen zugewandten Seite, des Behälters vorgesehen ist. Diese Ausgestaltung eignet sich besonders für kreisförmige Lüftungsschlitze, wie sie bei einer kreisförmigen Fondmitteldüse häufig anzutreffen sind.

Der Behälter 2 umfasst vorzugsweise einen abtrennbaren Deckel 10 und abtrennbare Seitenwände 11. Ferner ist dem Behälter 2 bevorzugt ein beweglicher Schieber 12 zugeordnet, mit dem sich die Öffnungen 7, 8 zumindest teilweise verschliessen lassen und hierüber die Beduftung regulieren lässt. Der Schieber 12 ist bevorzugt im Inneren des Behälters 2 angeordnet und weist je Öffnung 7, 8 eine Lasche 13 auf, die parallel zu der ihr zugeordneten Öffnung 7, 8 erstreckt und vor diese geschoben werden kann. Mit den Laschen 13 umgreift der Schieber 12 den Duftstoff 3 bzw. den Duftstift 5 teilweise. Der Schieber 12 weist vorzugsweise einen Griff 14 auf, mittels dem er manuell in Längsrichtung des Behälters 2 zum Schliessen der Öffnungen 7, 8 verschoben werden kann. Der Deckel 10 des Behälters 2 weist eine Öffnung 15 auf, durch die der Griff 14 hindurchragt, sodass ein Benutzer Zugriff auf den Griff 14 hat und über diesen den Schieber 12 bewegen kann.

Der Duftstoff 3 ist vorzugsweise, wie in den Figuren 1 bis 7 dargestellt, in Form eines auswechselbaren Duftstifts 5 vorgesehen, wobei der Duftstift 5 als Material einen Kompositwerkstoff aus zumindest einem Polymer, insbesondere einem Elastomer, und dem Duftstoff umfasst bzw. aus einem solchen Material besteht. Dabei ist das Polymer mit dem Duftstoff angereichert und fungiert als dessen Träger. Der Duftstift 5 weist bevorzugt mehrere Durchlassöffnungen 16 auf, durch die die aus dem Lüftungsschlitz austretende Luft strömen kann. Durch das Vorsehen der Durchlassöffnungen 16 wird die Oberfläche vergrössert, an welcher die aus dem Lüftungsschlitz austretende Luft mit dem Duftstoff in Kontakt gerät, was eine erhöhte Anreicherung der die Beduftungsvorrichtung 1 durchströmenden Luft mit Duftstoffmolekülen zur Folge hat. Die Durchtrittsöffnungen 16 sind vorzugsweise auf die Strömungsrichtung der Luft aus dem Lüftungsschlitz ausgerichtet. Ein derartiger Duftstift ist bereits aus der italienischen Patentanmeldung Nr. MI2009 A001982 und aus der Internationalen Patentanmeldung Nr. PCT/EP2010/067277 bekannt, auf die an dieser Stelle verweisen wird.

Alternativ kann der Duftstoff auch in Form eines Granulats vorgesehen sein, das in einem Käfig enthalten ist, welcher in dem Behälter 2 angeordnet ist.

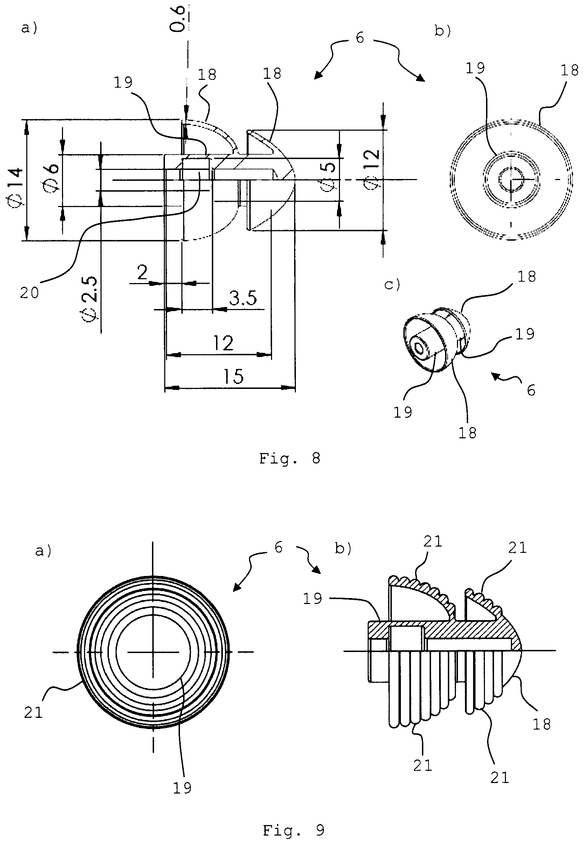

Wie zum Beispiel in den Figuren 6 und 8 dargestellt umfasst das Klemmteil 6 vorzugsweise zwei Schirme 18, die die Form eines elliptischen Paraboloids aufweisen, wobei der Extremwert, d.h. das Maximum, eines jeden elliptischen Paraboloids, vom Behälter 2 wegweist. Hierbei soll der Ausdruck "die Form eines elliptischen Paraboloids" auch die Form eines Stumpfes eines elliptischen Paraboloids umfassen. Alternativ können die einen oder mehreren Schirme die Aussenform eines Kegelstumpfes aufweisen. Die einen oder mehreren Schirme des Klemmteils können die Form eines Rotationsparaboloids bzw. eines Kegelstumpfes mit kreisförmiger Grundfläche haben. Alternativ können die einen oder mehreren Schirme die Form eines elliptischen Paraboloids mit ovaler Grundfläche bzw. eines Kegelstumpfes mit ovaler Grundfläche aufweisen. Die Schirme sind in Längsrichtung des Klemmteils 6 nacheinander angeordnet. Selbstverständlich kann das Klemmteil 6 auch nur einen Schirm oder mehr als zwei Schirme aufweisen. Die ein oder mehreren Schirme 18 des Klemmteils 6 passen dabei in die eingangs definierte Hüllform des Klemmteils 6 bzw. werden von dieser eingehüllt.

Je näher ein Schirm 18 im an den Behälter 2 angebrachten Zustand des Klemmteils 6 an dem Behälter 2 angeordnet ist, desto grösser ist vorzugsweise dessen Grundfläche im Vergleich zu den weiteren gegebenenfalls vorgesehenen Schirmen 18 des Klemmteils 6. Hierdurch wird das Einbringen des Klemmteils 6 in einen Lüftungsschlitz erleichtert. Figur 8 zeigt beispielhafte Dimensionen eines Klemmteils 6 mit zwei Schirmen 18, die jeweils die Form eines elliptischen Paraboloids aufweisen. Das Klemmteil 6 weist einen Schaft 19 auf, über welchen die Schirme 18 mittig miteinander verbunden sind.

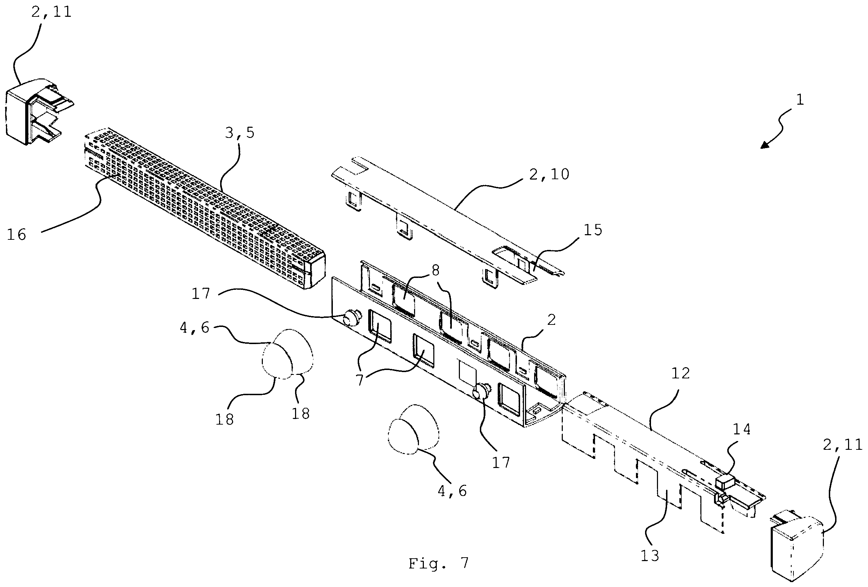

Die einen oder mehreren Klemmteile 6 werden vorzugsweise im Spritzgussverfahren hergestellt. Dabei können die Klemmteile 6 bereits direkt an den Behälter 2 angebracht werden. Alternativ kann jedes Klemmteil 6 über eine Schraubverbindung oder über eine Schnappverbindung an dem Behälter 2 befestigt sein. Eine Schnappverbindung kann beispielsweise mittels eines am Behälter 2 klemmteiligseitig für jedes Klemmteil 6 vorgesehen Haltenockens 17 realisiert sein, auf den das jeweilige Klemmteil 6 aufgesteckt wird (vergleiche Figuren 3 und 7). Das Klemmteil 6 weist behälterseitig eine Einbuchtung 20 auf, in welche der Haltenocken 17 beim Aufbringen/Aufstecken des Klemmteils 6 einrastet (vergleiche Figur 8).

Der Behälter 2 kann ferner zur Befestigung je Klemmteil 6 einen von ihm wegweisenden Stift aufweisen, auf den das jeweilige Klemmteil 6 aufgesteckt ist. Das Klemmteil 6 weist hierfür eine längliche Vertiefung auf, in die der Stift eingebracht wird, wobei der Durchmesser des Stiftes für einen festen Halt vorzugsweise etwas grösser als der Durchmesser der länglichen Vertiefung ist. Der Stift kann seitliche Ausbuchtungen aufweisen und die längliche Vertiefung kann seitliche Einbuchtungen aufweisen, wobei zur Befestigung eines Klemmteils 6 am Behälter 2 die seitlichen Ausbuchtungen des Stiftes in die seitlichen Einbuchtungen der länglichen Vertiefung eingebracht werden. Hierdurch wird eine Schnappverbindung erhalten. Alternativ kann der Stift seitliche Einbuchtungen und die längliche Vertiefung kann auf ihrer Innenseite nach innen ragende Ausbuchtungen aufweisen, wobei zur Befestigung eines Klemmteils 6 an dem Behälter 2 die nach innen ragenden Ausbuchtungen der länglichen Vertiefung in die seitlichen Einbuchtungen des Stiftes eingebracht werden, was ebenfalls zu einer Schnappverbindung führt. Der Stift und die längliche Vertiefung können zusätzlich oder alternativ mittels eines Klebstoffs miteinander verbunden sein.

Weiter kann jedes Klemmteil 6 behälterseitig ein elastisches Verbindungselement aufweisen, welches durch einen haltevorrichtungsseitigen Durchlass des Behälters 2 durchführbar ist und welches die haltevorrichtungsseitigen Wand des Behälters 2 hintergreift. Auch hierdurch wird eine Schnappverbindung erzielt.

Auf der Aussenseite der einen oder mehreren Schirme 18 können, wie Figur 9 zeigt, nach aussen weisende Noppen 21 vorgesehen sein, wobei sich jeder Noppen 21 in einer Querschnittsebene entlang des Umfangs des ihm zugeordneten Schirms 18 erstreckt und um den jeweiligen Schirm 18 herum geschlossen ist, also kreisförmig oder oval ist. Dies ermöglicht einen verbesserten Halt der Klemmteile 6 und damit der erfindungsgemässen Beduftungsvorrichtung 1 in einem Lüftungsschlitz im Innenraum eines Fahrzeugs. Die Noppen 21 können abgerundet sein (wie in Figur 9b) dargestellt), mit einer Spitze enden bzw. spitz zulaufend sein oder als Lamellen ausgeführt sein, wobei unter einer Lamelle eine dünne Scheibe bzw. ein dünnes Plättchen verstanden wird.

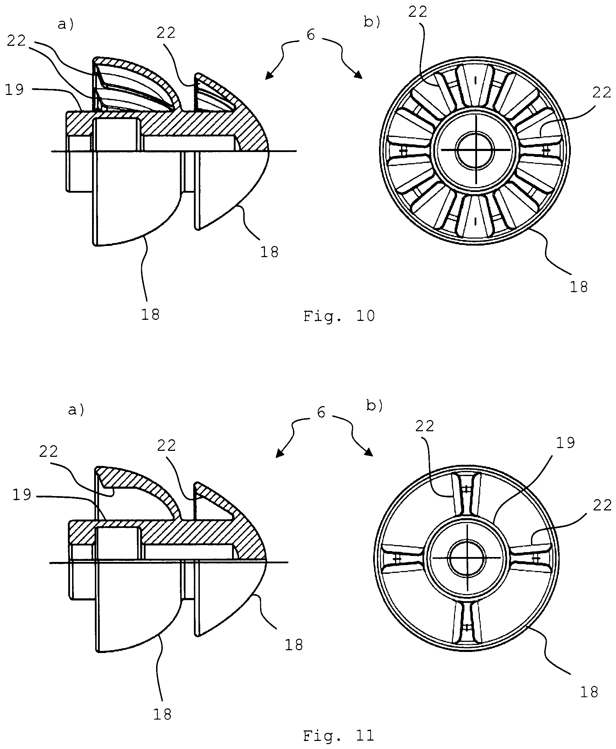

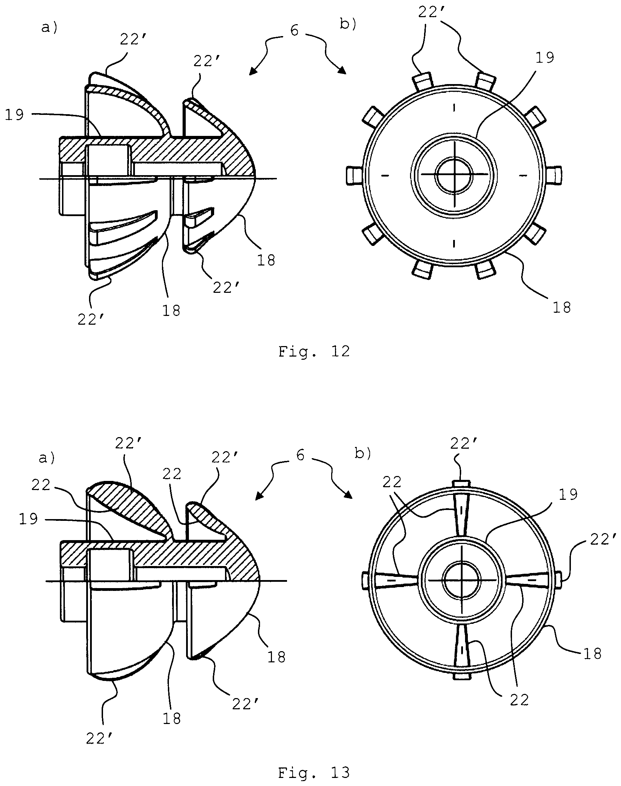

Den einen oder mehreren Schirmen 18 eines jeden Klemmteils 6 sind vorzugsweise Stützglieder 22, 22' zur Stützung der einen oder mehreren Schirme 18 zugeordnet (Figuren 10 bis 13), welche als Lamellen oder als Rippen ausgeführt sein können und welche sich in Längsrichtung des Klemmteils 6 erstrecken. Die Stützglieder können an der Innenseite eines Schirms 18 (Bezugszeichen 22 in Figuren 10 und 11), an dessen Aussenseite (Bezugszeichen 22' in Figur 12) oder an dessen Innen- und an dessen Aussenseite (Bezugszeichen 22 und 22' in Figur 13) angeordnet sein. Sind die Stützglieder 22, 22' an der Innen- und an der Aussenseite eines Schirms 18 angeordnet, wie in Figur 13 dargestellt, so liegen sie sich vorzugsweise gegenüber. Die Stützglieder 22, 22' erstrecken sich zumindest teilweise entlang der länglichen Ausdehnung des Schirms 18, dem sie zugeordnet sind.

Es sind vorzugsweise wenigstens vier Stützglieder 22, 22' auf zumindest einer Seite eines Schirms 18 vorgesehen, die in Querschnittsebene in einem 90 Grad-Winkel zueinander angeordnet sind (vergleiche Figuren 11 und 13). Es können jedoch auch mehr Stützglieder 22, 22' auf zumindest einer Seite eines Schirms 18 vorgesehen sein, wie die Figuren 10 und 12 zeigen, wobei der Winkel zwischen zwei benachbarten Stützgliedern 22, 22' bevorzugt gleich gross ist.

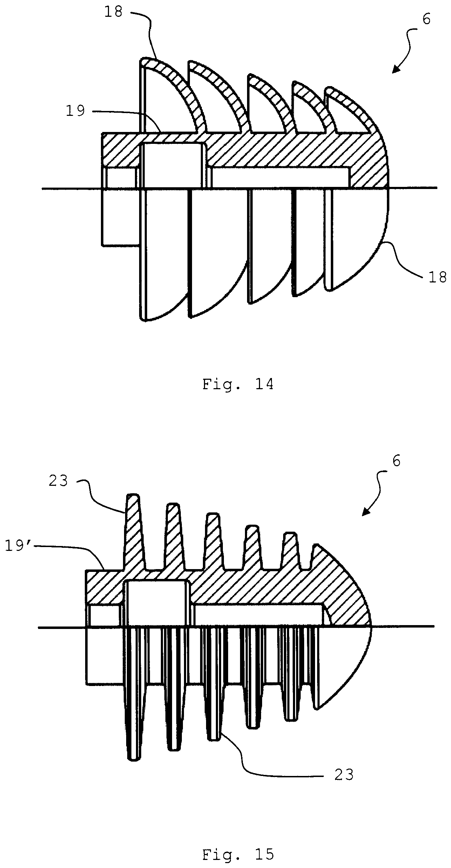

Figur 14 zeigt ein Klemmteil 6 mit beispielhaft fünf Schirmen 18, die in Längsrichtung aufeinanderfolgend angeordnet sind und die Form eines elliptischen Paraboloids aufweisen. Die Schirme 18 sind derart angeordnet, dass sie sich in Längsrichtung des Klemmteils 6 zumindest teilweise überlappen, was zu einer Erhöhung der Stabilität des Klemmteils 6 führt.

Gemäss alternativer, in Figur 15 dargestellter Ausführungsform weist jedes Klemmteil 6 der Beduftungsvorrichtung 1 ein oder mehrere Lamellen 23 auf, die sich in Querrichtung des Klemmteils 6 strahlenförmig erstrecken. Sind mehrere Lamellen 23 vorgesehen, so sind diese in Längsrichtung des Klemmteils 6 nacheinander angeordnet. Die Lamellen 23 sind über einen sich in Längsrichtung des Klemmteils 6 erstreckenden Schaft 19' miteinander verbunden. Es kann auch nur eine Lamelle 23 vorgesehen sein, die spiralförmig verlaufend an dem Schaft 19' angeordnet ist. Die Ausdehnung der einen oder mehreren Lamellen 23 in Querrichtung ist derart, dass sie in die eingangs definierte Hüllform des Klemmteils 6 passen bzw. von dieser eingehüllt wird.

Das wenigstens eine Klemmteil 6 ist vorzugsweise als separates Teil der Beduftungsvorrichtung 1 ausgeführt, das mit der Beduftungsvorrichtung 1 - bevorzugt lösbar - verbunden werden kann. Auf diese Weise können dem Benutzer mit einer Beduftungsvorrichtung mehrere Klemmteile unterschiedlicher Dimension, insbesondere unterschiedlichen Querschnitts, zur Verfügung gestellt werden, wobei die mehreren Klemmteile (beispielsweise drei Klemmteile) für unterschiedlich breite Lüftungsschlitze ausgestaltet sind. Der Benutzer kann dann vorteilhafterweise funktional dieselbe Beduftungsvorrichtung durch Austausch der einen oder mehreren Klemmteile sowohl in seinem Personenkraftwagen, als auch in seinem Transporter als auch in einem Lastkraftwagen einsetzen, welche typischerweise unterschiedlich breite Lüftungsschlitze aufweisen.

Während in der vorliegenden Anmeldung bevorzugte Ausgestaltungen bzw. Ausführungen der Erfindung beschrieben sind, ist klar darauf hinzuweisen, dass die Erfindung nicht auf diese beschränkt ist und auch in anderer Weise innerhalb des Umfangs der folgenden Ansprüche ausgeführt werden kann.

## Patentansprüche

1. Beduftungsvorrichtung für ein Fahrzeug mit einem Behälter (2) für einen Duftstoff (3), wobei der Behälter (2) für den Duftstoff (3) mit wenigstens einer Öffnung (7, 8) versehen ist, und einer Haltevorrichtung (4) zum Anbringen der Beduftungsvorrichtung (1) an einem Lüftungsschlitz im Innenraum eines Fahrzeugs, wobei die Haltevorrichtung (4) wenigstens ein Klemmteil (6) umfasst, wobei das wenigstens eine Klemmteil (6) aus elastischem Material ist und einen elliptischen oder kreisförmigen Querschnitt aufweist und derart ausgestaltet ist, dass es haltend in einen Lüftungsschlitz eines Fahrzeugs einbringbar ist, **dadurch gekennzeichnet, dass** das Klemmteil (6) einen oder mehrere Schirme (18) in der Form eines elliptischen Paraboloids oder mit der Aussenform eines Kegelstumpfes umfasst, die in Längsrichtung des Klemmteils (6) nacheinander angeordnet sind.

2. Beduftungsvorrichtung nach Anspruch 1, **dadurch gekennzeichnet, dass** die Hüllform des wenigstens einen Klemmteils (6) der Form eines elliptischen Paraboloids, eines Kegels oder eines Zylinders entspricht, wobei der Extremwert des Paraboloids bzw. die Spitze des Kegels von dem Behälter (2) für den Duftstoff (3) wegweist.

3. Beduftungsvorrichtung nach Anspruch 1 oder 2, **dadurch gekennzeichnet, dass** dem Behälter (2) ein Schieber (12) zugeordnet ist, mittels welchem die wenigstens eine Öffnung (7, 8) des Behälters (2) zumindest teilweise verschlossen werden kann.

4. Beduftungsvorrichtung nach einem der vorhergehenden Ansprüche, **dadurch gekennzeichnet, dass** ein oder mehrere Schirme (18) des Klemmteils (6) die Form eines Rotationsparaboloids oder die Aussenform eines Kegelstumpfes mit kreisförmiger Grundfläche haben.

5. Beduftungsvorrichtung nach einem der vorhergehenden Ansprüche, **dadurch gekennzeichnet, dass** auf der Aussenseite der einen oder mehreren Schirme (18) des Klemmteils (6) Noppen (21) vorgesehen sind.

6. Beduftungsvorrichtung nach Anspruch 5, **dadurch gekennzeichnet, dass** die Noppen (21) abgerundet sind, mit einer Spitze enden und/oder als Lamellen ausgeführt sind.

7. Beduftungsvorrichtung nach einem der vorhergehenden Ansprüche, **dadurch gekennzeichnet, dass** den einen oder mehreren Schirme (18) des Klemmteils (6) Stützglieder (22, 22') zur Stützung der einen oder mehreren Schirme (18) zugeordnet sind, wobei die Stützglieder (22, 22') insbesondere als Lamellen und/oder Rippen ausgeführt sind.

8. Beduftungsvorrichtung nach einem der vorhergehenden Ansprüche, **dadurch gekennzeichnet, dass** das Klemmteil (6) als separates Teil ausgeführt ist.

9. Beduftungsvorrichtung nach einem der vorhergehenden Ansprüche, **dadurch gekennzeichnet, dass** das Klemmteil (6) als Material Silikon, Gummi und/oder ein thermoplastisches Elastomer umfasst.

10. Beduftungsvorrichtung nach einem der vorhergehenden Ansprüche, **dadurch gekennzeichnet, dass** das Klemmteil (6) ein Material mit einer Härte im Bereich von 30 Shore-A bis 90 Shore-A umfasst.

11. Beduftungsvorrichtung nach einem der vorhergehenden Ansprüche, **dadurch gekennzeichnet, dass** der Behälter (2) länglich ausgeführt ist und wenigstens zwei Klemmteile (6) vorgesehen sind, wobei jeweils ein Klemmteil (6) in einem Endbereich des länglich ausgeführten Behälters (2) vorgesehen ist.

12. Beduftungsvorrichtung nach einem der vorhergehenden Ansprüche, **dadurch gekennzeichnet, dass** der Duftstoff (3) in Form eines Duftstifts (5) vorgesehen ist, wobei der Duftstift (5) als Material einen Kompositwerkstoff aus zumindest einem Polymer, insbesondere einem Elastomer, und dem Duftstoff (3) umfasst und mehrere Durchlassöffnungen (16) aufweist.

## Claims

1. Deodorization device for a vehicle with a container (2) for a deodorant (3), wherein the container (2) is provided with at least one opening (7, 8) for the deodorant (3), and a holding device (4) for mounting the deodorization device (1) onto a ventilation slot in the interior of a vehicle, wherein the holding device (4) comprises at least one clamping part (6), wherein the at least one clamping part (6) is made of an elastic material and has an elliptical or a circular cross-section and is configured so as to be able to be introduced in a holding manner into a ventilation slot of a vehicle, **characterized in that** the clamping part (6) comprises one or more panels (18) in the form of an elliptical paraboloid or with the external shape of a truncated cone arranged one after the other in the longitudinal direction of the clamping part (6).

2. Deodorization device according to claim 1, **characterized in that** the outer shape of the at least one clamping part (6) has the form of an elliptical paraboloid, equivalent to a cone or cylinder, wherein the extreme value of the paraboloid or apex of the cone faces away from the container (2) for the deodorant (3).

3. Deodorization device according to claim 1 or 2, **characterized in that** a slider (12) is assigned to the container (2), by means of which the at least one opening (7, 8) in the container (2) can be at least partially closed.

4. Deodorization device according to one of the preceding claims, **characterized in that** the one or more panels (18) of the clamping part (6) have the form of a paraboloid of revolution or the external shape of a truncated cone with a circular base.

5. Deodorization device according to one of the preceding claims, **characterized in that** protrusions (21) are provided on the exterior side of the one or more panels (18) of the clamping part (6).

6. Deodorization device according to claim 5, **characterized in that** the protrusions are rounded ending with a peak and/or embodied as ribs.

7. Deodorization device according to one of the preceding claims, **characterized in that** support members (22, 22') for the purpose of supporting the one or more panels (18) are assigned to the one or more panels (18) of the clamping part (6), wherein the support members (22, 22') are embodied in particular as lamellae and/or ribs.

8. Deodorization device according to one of the preceding claims, **characterized in that** the clamping part (6) is embodied as a separate part.

9. Deodorization device according to one of the preceding claims, **characterized in that** the clamping part (6) comprises silicon, rubber and/or a thermoplastic elastomer as material.

10. Deodorization device according to one of the preceding claims, **characterized in that** the clamping part (6) comprises a material with a hardness in the range of Shore 30A to Shore 90A.

11. Deodorization device according to one of the preceding claims, **characterized in that** the container (2) is effected in an elongated manner and provided with at least two clamping parts (6), wherein each clamping part (6) is provided at an end area of the elongated container (2).

12. Deodorization device according to one of the preceding claims, **characterized in that** the deodorant (3) is provided in the form of a scented stick (5), wherein the scented stick (5) comprises as material a composite made from at least one polymer, in particular an elastomer, and the deodorant (3), and said scented stick has multiple outlet openings (16).

## Revendications

1. Dispositif de désodorisation pour un véhicule avec un récipient (2) pour un déodorant (3), le récipient (2) étant doté d'au moins une ouverture (7, 8) pour le déodorant (3), et un dispositif de retenue (4) pour monter le dispositif de désodorisation (1) sur une fente de véhicule à l'intérieur d'un véhicule, le dispositif de retenue (4) comprenant au moins une pièce de serrage (6), l'au moins une pièce de serrage (6) étant constitué d'un matériau élastique et ayant une coupe transversale elliptique ou circulaire et étant configurée de manière à pouvoir être introduite de manière supportée dans une fente de ventilation d'un véhicule, **caractérisé en ce que** la pièce de serrage (6) comprend un ou plusieurs panneaux (18) en forme d'un paraboloïde elliptique ou avec une forme externe d'un cône tronqué arrangé l'un après l'autre en direction longitudinale de la pièce de serrage (6).

2. Dispositif de désodorisation selon la revendication 1, **caractérisé en ce que** la forme extérieure de l'au moins une pièce de serrage (6) a la forme d'un paraboloïde elliptique, équivalent à un cône ou un cylindre, la valeur extrême du paraboloïde ou l'apex du cône étant opposé au récipient (2) pour le déodorant (3).

3. Dispositif de désodorisation selon la revendication 1 ou 2, **caractérisé en ce qu'**un glisseur (12) est attribué au récipient (2), à l'aide duquel l'au moins une ouverture (7, 8) dans le récipient (2) peut être fermé au moins partiellement.

4. Dispositif de désodorisation selon l'une des revendications précédentes, **caractérisé en ce que** l'un panneau ou les plusieurs panneaux (18) de la pièce de serrage (6) ont la forme d'un paraboloïde de révolution ou la forme externe d'un cône tronqué avec une base circulaire.

5. Dispositif de désodorisation selon l'une des revendications précédentes, **caractérisé en ce que** des protubérances (21) sont prévues du côté extérieur de l'un panneau ou les plusieurs panneaux (18) de la pièce de serrage (6).

6. Dispositif de désodorisation selon la revendication 5, **caractérisé en ce que** les protubérances sont arrondies et finissent avec une pointe et/ou en forme de nervure.

7. Dispositif de désodorisation selon l'une des revendications précédentes, **caractérisé en ce que** des membres de support (22, 22') pour supporter l'un panneau ou les plusieurs panneaux (18) sont attribués à l'un panneau ou aux plusieurs panneaux (18) de la pièce de serrage (6), les membres de support (22, 22') étant particulièrement en forme de lamelles et/ou de nervures.

8. Dispositif de désodorisation selon l'une des revendications précédentes, **caractérisé en ce que** la pièce de serrage (6) est en forme de pièce séparée.

9. Dispositif de désodorisation selon l'une des revendications précédentes, **caractérisé en ce que** la pièce de serrage (6) comprend comme matériau de la silicone, gomme et/ou un élastomère thermoplastique.

10. Dispositif de désodorisation selon l'une des revendications précédentes, **caractérisé en ce que** la pièce de serrage (6) comprend un matériau avec une rigidité comprise entre Shore 30A et Shore 90A.

11. Dispositif de désodorisation selon l'une des revendications précédentes, **caractérisé en ce que** le récipient (2) est effectué de manière allongée et doté d'au moins deux pièces de serrage (6), chaque pièce de serrage (6) étant prévue dans une région d'extrémité du récipient allongé.

12. Dispositif de désodorisation selon l'une des revendications précédentes, **caractérisé en ce que** le déodorant (3) est prévu en forme d'un stick parfumé (5), le stick parfumé (5) comprenant comme matériau un composite constitué d'au moins un polymère, particulièrement un élastomère, et le déodorant (3), et **en ce que** ledit stick parfumé a une pluralité d'ouvertures de sortie (16).
